# FASCICULE DE BREVET EUROPEEN

(11) **EP 4 135 785 B1**
(45) Date de publication et mention de la délivrance du brevet: **23.07.2025**
(21) Numéro de dépôt: 21722110.0
(22) Date de dépôt: 16.04.2021
(51) Int. Cl.: A61L 27/18, A61L 27/34, A61L 27/36

(54) **DISPOSITIF MEDICAL COMPRENANT UNE MATRICE BIOLOGIQUE ACELLULAIRE ET AU MOINS UN POLYMERE**
MEDIZINISCHE VORRICHTUNG MIT EINER ZELLFREIEN BIOLOGISCHEN MATRIX UND MINDESTENS EINEM POLYMER
MEDICAL DEVICE COMPRISING A CELL-FREE BIOLOGICAL MATRIX AND AT LEAST ONE POLYMER

(30) Priorité: 17.04.2020 FR 2003871
(43) Date de publication de la demande: 22.02.2023
(73) Titulaire: PH Tech, 33550 Le Tourne (FR)
(72) Inventeur: PERES, Anthony, 17139 DOMPIERRE SUR MER (FR); HOFMANSKI, Guillaume, 61250 MIEUXCE (FR)
(74) Mandataire: Aquinov
(86) Numéro de dépôt international: PCT/EP2021/059944
(87) Numéro de publication internationale: WO 2021/209615

(56) Documents cités:
- WO-A1-2016/014119
- WO-A1-2016/094166
- WO-A2-2008/070428

## Description

### Domaine technique

L'invention concerne un dispositif médical particulier comprenant une matrice biologique, ainsi que son procédé de fabrication et son utilisation.

### Etat de l'art

Beaucoup d'applications chirurgicales requièrent le renforcement des tissus mous avec des éléments conjuguant résistance mécanique, souplesse et compatibilité biologique.

A cet effet, les matrices biologiques sont de plus en plus utilisées pour la fabrication de dispositifs médicaux du fait de leur compatibilité biologique. Toutefois, dans le cas de chirurgies septiques ou potentiellement septiques, les implants biologiques peuvent être digérés et dégradés à cause d'une charge enzymatique (collagénases notamment) trop importante ce qui rend leur utilisation inadaptée pour de nombreuses applications.

Les documents WO 2016/014119 A1 et WO 2016/094166 A1 divulguent un dispositif médical comprenant une matrice extra cellulaire acellulaire et un polymère choisi parmi les hydroxybutyrates et autres PHAs. Le polymère est utilisé comme revêtement protecteur (coating) afin de contrôler la dégradation enzymatique de la matrice.

Il existe par conséquent un besoin pour des implants biologiques qui présentent une intégrité mécanique et un renfort optimal durant la reconstruction tissulaire même en cas d'infections. L'objectif de l'invention est de répondre à ce besoin.

### Résumé de l'invention

A cet effet l'invention a pour objet un dispositif médical comprenant au moins une matrice biologique acellulaire et au moins un polymère, en particulier au moins matrice biologique acellulaire recouverte partiellement ou totalement par au moins une couche comprenant au moins un polymère, comme définit dans les revendications.

Avantageusement, la présence d'au moins un polymère avec la matrice biologique acellulaire permet de rendre la matrice biologique résistante aux infections, tout en conservant les qualités de la matrice biologique en termes de résistance mécanique, souplesse et comptabilité biologique. Il peut ainsi être utilisé pour des applications médicales en particulier en chirurgie.

L'invention a également pour objet un procédé de fabrication d'un tel dispositif médical. Le procédé comprend la mise en œuvre des étapes suivantes :
- préparation d'une matrice biologique cellulaire de façon à permettre l'adhérence d'une solution comprenant au moins un polymère, par un traitement de surface chimique et/ou mécanique et/ou électrochimique et/ou physique, et
- enduction partielle ou totale de la matrice biologique acellulaire avec une solution comprenant au moins un polymère.

D'autres caractéristiques et avantages ressortiront de la description en détail de l'invention qui va suivre.

### Description détaillée de l'invention

### Définitions

Par matrice biologique « acellulaire » au sens de l'invention on entend une matrice biologique dans laquelle les éléments cellulaires ont été éliminés par un procédé de décellularisation dans l'objectif de détruire et/ou enlever les cellules et leurs composants de la matrice extracellulaire de la matrice biologique tout en maintenant sa structure et ses propriétés. En effet, pour qu'une matrice biologique puisse être implantée chez un receveur, elle doit être décellularisée de façon à diminuer son immunogénicité.

Par « Allograft » ou « allogreffe » au sens de l'invention on entend une matrice biologique, un greffon, provenant d'un donneur faisant partie de la même espèce biologique que le receveur.

Par « Implant » au sens de l'invention on entend un dispositif médical utilisé en chirurgie.

Par « matrice biologique » au sens de l'invention on entend un biomatériau dérivé de l'espèce humaine ou animale.

Par « P4HB » au sens de l'invention on entend un PHA particulier qui signifie Poly-4-hydroxybutyrate. Il s'agit d'un homopolymère d'unité 4-hydroxybutyrate.

« Peel test » est utilisé ici en référence à un test permettant de déterminer la force d'adhésion entre 2 matériaux. Chaque matériau est placé dans des mâchoires pneumatiques à une pression donnée puis séparé à vitesse constante comme précisé dans les exemples.

Par « PHAs » au sens de l'invention on entend des polyhydroxyalcanoates qui sont des polyesters biodégradables.

Par « solution » au sens de l'invention on entend un mélange homogène résultant de la dissolution d'un ou plusieurs soluté(s) dans un solvant.

Par « Suture Retention Force » (« force de rétention de suture ») ou «Suture Retention Strength » (« Tension de rétention de suture ») au sens de l'invention on entend un test permettant de déterminer la force (N) nécessaire pour tirer une suture hors d'un spécimen.

Par « Uniaxial tensile Strength » (« résistance uniaxiale à la traction ») au sens de l'invention on entend un test permettant de déterminer la tension avant rupture du spécimen testé. Les propriétés mesurées sont Ultimate Tensile Strength (résistance maximale à la traction), la force de rupture et l'élongation à la rupture.

Par « Viscosité » au sens de l'invention on entend une propriété de résistance à l'écoulement d'un fluide pour un écoulement sans turbulence.

Par « Xenograft » ou « xenogreffe » au sens de l'invention on entend une matrice biologique, un greffon, provenant d'un donneur faisant partie d'une espèce biologique différente de celle du receveur.

### Dispositif médical

Selon un premier aspect, l'invention vise donc un dispositif médical comprenant :
- au moins une matrice biologique acellulaire, et
- au moins un polymère, caractérisé en ce que la matrice biologique acellulaire a été préparée avant enduction du polymère, de façon à permettre l'adhérence d'une solution de polymère, par un traitement de surface chimique et/ou mécanique et/ou électrochimique et/ou physique.

Les matrices biologiques acellulaires regroupent une large classe de biomatériaux extraient de greffons d'origines diverses.

Préférentiellement la matrice biologique dans le dispositif médical selon l'invention, est d'origine humaine et/ou animale.

Selon un mode de réalisation particulièrement adapté il s'agit d'une matrice biologique choisie parmi les matrices biologiques d'origine porcine, bovine, équine, caprine, de poissons et leurs mélanges.

La matrice biologique selon l'invention peut être choisie parmi toutes les matrices biologiques animales et/ou humaines, préférentiellement parmi l'une des matrices biologiques suivantes : derme, sous-muqueuse intestinale, aorte, vessie, membrane amniotique, péritoine, péricarde, dure-mère, tendons, os, cartilage et leurs mélanges.

La matrice biologique du dispositif médical selon l'invention est acellulaire. Il existe de nombreux procédés connus permettant d'obtenir une matrice biologique acellulaire. Les procédés utilisés peuvent être enzymatiques et/ou à base de solutions chimiques et/ou s'appuyant sur des procédés mécaniques. Le procédé utilisé doit être un procédé permettant d'obtenir une matrice biologique acellulaire apte à être utilisée en chirurgie en particulier pour la reconstruction des tissus mous.

La matrice biologique acellulaire selon l'invention est préférentiellement une matrice biologique acellulaire qui présente au moins l'une des caractéristiques suivantes, encore plus préférentiellement toutes :
- « Uniaxial Tensile Strength » supérieur ou égal à 5N/mm2 pour un spécimen de 5 mm x 50 mm avec une épaisseur prise en compte dans le calcul. Chaque extrémité est attachée dans des mords pneumatiques dans le sens de la longueur à 1 cm de chaque bord. La vitesse d'écartement des bords est de 30 mm/min. « Uniaxial Tensile Strenght » est reporté en divisant la force maximale (N) / (5 (mm) x épaisseur (mm)).
- « Suture Retention Force » supérieure ou égale à 5N pour un échantillon de 1cm x 1cm en utilisant une suture appropriée de (type 4-0 polypropylène) enfilée à 3 mm du bord de l'échantillon en son centre, le côté opposé étant clampé dans un mord pneumatique à 5 mm approximativement. Les deux extrémités du fil de suture sont clampées dans le mord inférieur. La vitesse de séparation des mords est de 20mm/min. La force (N) maximum est retenue.

Préférentiellement, la matrice biologique utilisée présente les spécifications définies dans les standards applicables (« USP official monographs » dernière version, ASTM) selon le type de matrice biologique utilisée.

La matrice biologique acellulaire du dispositif selon l'invention peut être de forme variée. Préférentiellement elle :
- est sèche, de façon préférée elle présente un taux d'humidité résiduel compris entre 10% et 18%, et/ou
- elle présente un aspect de surface améliorant l'adhérence d'au moins un polymère, en particulier l'adhérence d'au moins une solution de polymère(s). Cet aspect de surface est obtenu par un traitement/ une préparation de la matrice biologique acellulaire avant enduction, notamment par traitement de surface chimique et/ou mécanique et/ou électrochimique et/ou physique.

Le polymère présent dans le dispositif médical selon l'invention peut être tout type de polymère adapté à une utilisation comme dispositif médical et en particulier comme implant chirurgical.

Le ou les polymères présents dans le dispositif selon l'invention sont préférentiellement choisis parmi les polymères suivants : poly(glycolides), poly(lactide-co-glycolides); acide polylactique, acide polyglycolique, poly(acide lactique co acides glycolique), polycaprolactones, poly(orthoesters), polyanhydrides, poly(phosphazenes), polyhydroxyalcanoates (incluant notamment P4HB et poly-3-hydroxybutyrate-co-3- hydroxy valerate (PHBV)), polyesters, poly(lactide-co-caprolactones), polycarbonates, tyrosine polycarbonates, polyamides, polyesteramides, poly(dioxanones), poly(alkylene alkylates), polyethers, polyvinyl pyrrolidones ou PVP, polyurethanes, polyetheresters, polyacetals, polycyanoacrylates, poly(oxyethylene)/poly(oxypropylene) copolymers, polyacetals, polyketals, polyphosphates, polyphosphoesters, polyalkylene oxalates, polyalkylene succinates, acides polymaléique, chitine, chitosan, et leurs mélanges.

Selon un mode de réalisation particulièrement adapté, le dispositif selon l'invention comprend au moins un PHA et encore plus préférentiellement un PHA choisi parmi au moins le P4HB, les copolymères du P4HB et leurs mélanges. Les PHAs constituent une famille de matériaux produits par de nombreux micro-organismes. On peut citer par exemple le brevet US 6,316,262 de la société Meta-bolix, Inc. Of Cambridge MA, USA, qui décrit une méthode permettant l'obtention d'un système biologique permettant la production de polymères de polyhydroxyalcanoate contenant du 4-hydroxya-cides. Les brevets US 6,245,537, US 6.623.748, US 7,244,442 et US 8,231,889 décrivent également des méthodes d'élaboration de PHAs adaptés à une utilisation médicale et à des dispositifs médicaux selon l'invention.

Préférentiellement le PHA et en particulier le P4HB et/ou ses copolymères présentent un niveau bas d'endotoxines, en particulier ils permettent d'avoir taux inférieur à 20 EU par dispositif médical. Préférentiellement, le dispositif comprend au moins une matrice biologique acellulaire recouverte partiellement ou totalement par au moins une couche comprenant au moins un polymère.

Le dispositif selon l'invention peut comprendre une ou plusieurs matrices biologiques acellulaires, une ou plusieurs couches de polymère(s) et éventuellement d'autres constituants. Le dispositif médical selon l'invention peut comprendre par exemple au moins une matrice biologique acellulaire recouverte partiellement ou totalement par au moins deux couches comprenant au moins un polymère.

Selon un mode de réalisation particulier, le dispositif médical est constitué exclusivement par une matrice biologique acellulaire recouverte partiellement ou totalement par une couche comprenant au moins un polymère.

Selon un autre mode de réalisation particulier, le dispositif médical est constitué exclusivement par une matrice biologique acellulaire recouverte partiellement ou totalement par deux couches comprenant au moins un polymère.

La présence du ou des polymères dans le dispositif médical permet un renfort optimal durant la reconstruction tissulaire supportée par la matrice biologique et cela même en cas d'infection.

La ou les couches de polymères peu(ven)t comprendre un ou plusieurs canaux qui permettent d'incorporer lors de l'utilisation en chirurgie, des éléments favorisant une reconstructions optimale des tissus dans lequel le dispositif est utilisé comme implant (PRP, cellules souches, antibiotiques, etc). Ces canaux peuvent être circulaires ou non circulaires. Ils ont préférentiellement une surface interne comprise entre 0,007 mm² et 0,8mm². Ils peuvent être obtenus par impression d'une forme dans la matrice biologique par pressage avant enduction de la matrice biologique. La forme étant alors retirée après enduction et séchage.

### Procédé de fabrication

Le dispositif médical selon l'invention peut être obtenu par tout procédé adapté. En particulier l'invention a pour objet un procédé de fabrication d'un dispositif médical comprenant la mise en œuvre des étapes suivantes :
- préparation d'une matrice biologique cellulaire de façon à permettre l'adhérence d'une solution comprenant au moins un polymère, par un traitement de surface chimique et/ou mécanique et/ou électrochimique et/ou physique, et
- enduction partielle ou totale de la matrice biologique acellulaire avec une solution comprenant au moins un polymère.

L'étape de préparation de la matrice biologique cellulaire consiste en un traitement de surface chimique et/ou mécanique et/ou électrochimique et/ou physique. Il peut s'agir par exemple d'un traitement par abrasion et/ou fraisage et/ou microtexturation et/ou laser et/ou UV.

La matrice biologique doit être préparée de façon à permettre l'adhérence d'une solution de polymère(s). La matrice biologique convenablement préparée peut avoir différentes formes (rondes, carrés, circulaires, irrégulières ...), plane ou en relief, avec des canaux, avec l'une des surfaces texturées, être d'épaisseur régulière ou irrégulière.

Dans un mode de réalisation préférée, la matrice biologique doit être sèche ou séchée de façon à présenter un taux d'humidité résiduel de l'ordre de 10% à 18%. Le taux d'humidité résiduel est préférentiellement mesuré à l'aide d'un dessiccateur type Halogen Moisture Analyzer de marque Mettler Toledo.

Une technique de séchage utilisée est préférentiellement celle du « Loss on Drying » (perte au séchage) décrite dans l'USP 41 (« scaffold bovine dermis » derme bovin) :
- 1- une coupelle vide en aluminium est positionnée dans l'appareil et la tare est réalisée,
- 2 la coupelle est remplie avec un échantillon de 1,0 g +/- 0,2 g coupé en morceaux de 4 mm²,
- 3 le programme de chauffe à 130°C est lancé
- 4 lorsque le poids ne varie plus sur un temps donné, le résultat est affiché en %.

Une autre méthode de séchage par étuve conventionnelle peut également être utilisée. Dans ce cadre une coupelle en aluminium préalablement pesée à vide est remplie avec un échantillon de 5,0 g +/- 0,2 g coupé en morceaux 4 mm². L'ensemble est placé à 100°C pendant 16h. L'ensemble est alors pesé et le « loss on drying » (perte au séchage) est calculé :
- matière sèche % = [(poids de l'extrait sec + coupelle (g) - poids de la coupelle (g))/g de l'échantillon] x 100
- humidité % = 100 - matière sèche %.

La matrice biologique préparée est ensuite utilisée comme support pour l'enduction d'une solution de polymères.

Préférentiellement la solution comprenant au moins un polymère a été préalablement obtenue par solubilisation du ou des polymère(s) sec(s) dans au moins un solvant, préférentiellement au moins un solvant polaire.

Le ou les polymères doivent en effet préférentiellement être convertis en une solution propre à permettre l'enduction en utilisant le solvant adéquat.

Lorsque le polymère est un PHA et en particulier le P4HB et/ou un de ses copolymères, le solvant est préférentiellement choisi parmi les solvants polaires suivants : dichlorométhane, chloroforme, tetrahydrofurane, dioxane, acétone et leurs mélanges.

Dans un mode de réalisation préféré, le P4HB est solubilisé dans une solution d'acétone, préférentiellement selon un ratio 5% à 25% (w/w). Les quantités respectives sont mises en présence, l'ensemble est chauffé et maintenu à une température inférieure au point d'ébullition de l'acétone (approximativement 56°C) jusqu'à dissolution complète du P4HB et obtention de la viscosité désirée au moins 10%, plus préférablement 15% et encore plus préférablement 20% (w/w). Préférentiellement, après solubilisation du ou des polymère(s) dans un solvant, la solution de polymère(s) est dégazée et/ou débullée pour purger le mélange de bulles d'air. De façon préférée la solution est placée sous vide (minimum -1 bar) pendant le temps nécessaire à un dégazage / débul-lage complet.

L'étape d'enduction de la matrice biologique acellulaire par une solution de polymère(s) est préférentiellement réalisée à une température inférieure ou égale à la température de dénaturation du collagène. De façon particulièrement adaptée, l'enduction est réalisée à une température comprise entre 10 et 60°C, préférentiellement entre 10 et 50°C, encore plus préférentiellement entre 20 et 50°C.

L'enduction peut être réalisée par tout moyen adapté, préférentiellement par « solvant casting » (enduction par coulage), « spray coating » (enduction par vaporisation), « dip coating » (enduction par immersion).

Avantageusement, le procédé selon l'invention permet l'application directe d'une solution de polymère(s) à la concentration désirée sur une matrice biologique acellulaire préalablement préparé et il n'est pas nécessaire de préalablement fabriquer un film ou une feuille de polymère pour ensuite le disposer sur le support afin de permettre l'adhésion des éléments entre eux par chauffage. Lorsqu'elle est réalisée par « solvent casting », l'enduction peut être réalisée avec différentes technologies : « knife » (couteau), « double side » (double face), commabar, « case knife » (couteau), « engraved roller » (rouleau engravé), « 2 roller » (2 rouleaux), « 3 roller combi » (combi 3 rouleaux), « micro roller » (micro rouleau), « 5 roller » (5 rouleaux), « reverse roller » (rouleau reversé), « rotary screen » (rotation), « dipping » (immersion), « slot Die » (fente), « curtain coating » (enduction rideau), « hotmelt slot die » (fondoir). La méthode la plus simple repose sur l'utilisation d'un « casting knife » (couteau de coulée) type Elcometer. La matrice biologique est disposée sur une table, en fonction de l'épaisseur souhaitée et de la surface à traiter la quantité nécessaire de solution de polymère(s) est disposée sur la matrice biologique acellulaire. Le « Gardener Knife » (couteau de jardinier) est alors déplacé sur la matrice biologique acellulaire afin d'uniformiser l'enduction sur l'implant. Le « Gardener Knife » a préalablement été ajusté à une certaine hauteur. Dans le but d'automatiser l'opération une « coating machine » (machine de revêtement) peut être utilisée. La solution de polymère(s) est pompée à travers un « slot die » (matrice de fente) pour être appliquée sur l'implant biologique plan en mouvement. Dans un mode de réalisation préférée la largeur du slot die est de 600 mm, la vitesse de progression de l'implant est de 1 - 10 m/min. La vitesse de pompage, la vitesse de progression, la largeur du « slot die » et la concentration de la solution peuvent être ajustées afin d'obtenir l'implant avec une enduction d'épaisseur et de largeur souhaitées.

Lorsque l'enduction est réalisée par « spray coating » ou pulvérisation, la solution de polymère(s) est pompée jusqu'à une buse qui projette des gouttelettes sur la surface à traiter. Cette technique est particulièrement avantageuse pour le traitement de matrices biologiques qui présentent des formes 3D (exemple : implant biologique ayant une forme hémisphérique, ovoide, tubulaire, en forme d'implants mammaires anatomiques, etc.).

Lorsque l'enduction est réalisée par « Dip coating » (revêtement par immersion), ou par trempage, la pièce à traiter est trempée dans une matière dissoute, fondue, ramollie ou en poudre fluidisée afin de la recouvrir d'une couche de cette matière. Cette technique est particulièrement avantageuse pour le traitement d'implants biologiques en relief et plan.

Après enduction, le dispositif médical obtenu est composé d'une couche de matrice biologique enduite partiellement ou totalement d'au moins un polymère en solution, et l'ensemble étant ensuite séché puis préférentiellement pressé pour obtenir une épaisseur uniforme.

Dans un mode de réalisation préféré les matrices biologiques acellulaires enduites par la solution de polymère(s) sont placés dans une étuve ou un four ou une enceinte de chauffe afin de permettre l'évaporation complète du solvant. A une température entre 0°C et 50°C, comprise entre 15 et 40°C, préférablement 30°C plus ou moins 5°, de façon à éviter une évaporation trop rapide et une déformation de la matrice biologique.

Selon une variante, les matrices biologiques acellulaires enduites par la solution de polymère(s), éventuellement avant ou après séchage, sont pressés dans une presse hydraulique sur une durée de 30 à 60 s, entre 6 bars et 200 bars, notamment entre 50 et 200 bars et à une température entre 50°C et 190°C, notamment entre 50°C et 100°C. Les matrices biologiques planes (et par conséquent les dispositifs médicaux selon l'invention plans) sont traitées dans une presse hydraulique entre 2 plateaux. Les matrices biologiques (et par conséquent les dispositifs médicaux selon l'invention) en relief (type implants biologiques traités par ladite méthode en forme d'implants mammaires de formes anatomiques ou hémisphériques) peuvent être pressés dans des moules disposant des empreintes souhaitées (type « molding machine for fabric cup » (presse à bonnets soutien-gorge).

### Utilisations

Le dispositif médical selon l'invention peut être utilisé pour toute application médicale en particulier comme implant en particulier en chirurgie. Il peut être utilisé en tant que tel ou transformé pour être utilisé en chirurgies.

En particulier et de façon non limitative les dispositifs médicaux selon l'invention peuvent être utilisés pour les applications suivantes : réparation, régénération et remplacement des tissus mous et durs, dispositif de cicatrisation, bandage, patch, pansement, pansement pour brûlures, pansement pour ulcère, substitut cutané, hémostatique, dispositif de reconstruction trachéale, dispositif de sauvetage d'organes, substitut dural, patch dural, nerf guide, dispositif de régénération ou de réparation nerveuse, dispositif de réparation de hernie, maille de hernie, bouchon de hernie, dispositif de support temporaire de plaie ou de tissu, échafaudage d'ingénierie tissulaire, dispositif de réparation / régénération de tissu guidé, dispositifs de fixation pour mailles, membrane anti-adhérence, adhérence barrière, membrane de séparation des tissus, membrane de rétention, élingue, dispositif de reconstruction du plancher pelvien, dispositif de suspension urétrale, dispositif de traitement de l'incontinence urinaire, dispositif de réparation de la vessie, dispositif de gonflement ou de remplissage, dispositif de réparation de la coiffe des rotateurs, dispositif de réparation du ménisque, dispositif de régénération du ménisque, membrane de régénération tissulaire guidée pour les tissus parodontaux, dispositif d'anastomose, dispositif ensemencé de cellules, dispositif d'encapsulation cellulaire, dispositif de libération contrôlée, dispositif d'administration de médicaments, dispositif de chirurgie plastique, dispositif de lifting mammaire, dispositif de mastopexie, dispositif de reconstruction mammaire, dispositif d'augmentation mammaire, dispositif de réduction mammaire, appareils de reconstruction mammaire après mastectomie avec ou sans implants mammaires, appareil de rhinoplastie.

L'invention est à présent illustrée par des exemples et des résultats d'essais.

### Exemples et résultats d'essais

### Exemple 1 : traitement de la matrice biologique acellulaire

Une matrice dermique acellulaire plane est placée sur une machine-outil à commande numérique. Une fraise carbure diamètre 5 mm est montée sur la machine-outil. Une vitesse de rotation de 20000 à 40000 tours/min est utilisée avec une avance de 2 m/min. La profondeur est variable en fonction de l'épaisseur finale souhaitée. Le surfaçage peut être total ou ne concerner qu'une partie de l'implant de manière à délimiter des formes.

### Exemple 2 : traitement de la matrice biologique acellulaire

Une matrice dermique acellulaire plane est placée sur une machine brosseuse / cardeuse. La surface de l'implant est ainsi traitée par des cardes de diamètre souhaitée (par exemple 105 mm) composées de fils métalliques (de diamètre par exemple 0,2 mm).

### Exemple 3 : traitement de la matrice biologique acellulaire

Une matrice dermique plane est placée sur une ponceuse type Mercier-Turner. La surface de l'implant est poncée par un abrasif grain 240.

Une section de l'implant abrasée (6 cm x 6 cm) est enduite à approximativement 0,02g/cm² de P4HB et est pressée pendant 60s à 50 bars de pression et 50°C (plateaux presse préalablement chauffée). Un « T peel » test est réalisé. Des spécimens de 2 cm x 6 cm sont découpés. L'enduction de P4HB est séparée de l'implant biologique sur une section de 1,5 cm x 2 cm. Les 2 morceaux ainsi séparés sont placés dans les mords pneumatiques (45 psi). La section de l'implant biologique enduite est séparée à une vitesse de 25 mm/min. La « T-peel » force est mesurée sur une largeur normalisée de 20 mm et sur une moyenne de 5 pics (charges). Sur les 3 spécimens testés, l'adhésion était plus forte de sorte que le test n'a pas permis de peler la couche enduite.

### Exemple 4 : traitement de la matrice biologique acellulaire

Une matrice dermique acellulaire est traitée avec un champ électrique haute fréquence type Telea Biotech (4-64 MHz) de façon à créer des cavités et/ou perforations de 0,6mm de diamètre.

### Exemple 5 : traitement de la matrice biologique acellulaire

Une matrice dermique acellulaire est traitée comme précisé dans l'exemple 1. Une fraise carbure de 2 mm est montée sur la machine-outil afin de dessiner dans la matrice des canaux en forme sinusoïdale sur la longueur de la matrice. Vitesse et avance sont similaires à l'exemple 1. Les canaux sont répétés à intervalle régulier sur la largeur de façon à couvrir une partie ou la totalité de l'implant. La profondeur des canaux est fonction du diamètre final désiré. Dans un autre mode de réalisation une empreinte peut être fabriquée de façon à imprimer par pressage les canaux dans la matrice.

### Exemple 6 : exemple de procédé d'enduction par « solvent casting »

Une matrice dermique acellulaire traitée selon l'exemple 1 de dimensions 2 cm x 6 cm soit 12 cm² est disposé sur une table. La quantité souhaitée de P4HB étant de 0,0164 g/cm², une solution P4HB/acétone à 18% (w/w) est préparée. La quantité nécessaire est prélevée et déposée sur l'implant. Le « casting knife » est alors déplacé par translation sur l'implant afin d'uniformiser l'épaisseur de l'enduction. Dans le but d'automatiser l'opération une « coating machine » peut être utilisée. Une solution acétone/P4HB est préparée afin d'alimenter la machine. La solution est pompée à travers un « slot die » pour être appliquée sur l'implant biologique plan en mouvement. Dans un mode de réalisation préférée la largeur du « slot die » est de 600 mm, la vitesse de progression de l'implant est de 1 - 10 m/min.

### Exemple 7 : exemple de procédé de dip coating

Une matrice biologique acellulaire traitée selon l'exemple 4 de 4 cm x 6 cm est immergé par une machine (l'implant est disposé sur une traverse verticale) dans un bac contenant une solution acétone/P4HB à la concentration souhaitée. La matrice biologique est alors extraite du bac par un mouvement vertical ascendant à une vitesse de 25mm/min. La densité de P4HB obtenue par l'enduction est alors de 0,02 à 0,04 g/cm². Les paramètres (taille de la pièce à traiter, vitesse de la traverse, concentration de la solution) sont appréhendables par l'homme de l'art afin d'obtenir l'enduction souhaitée in fine.

### Exemple 8 : exemple de séchage et pressage et caractéristiques des dispositifs médicaux obtenus

Une matrice dermique traitée selon l'exemple 1 est enduite à approximativement 0,03g/cm² de P4HB et est pressée pendant 30s à 100 bars de pression et 50°C (plateaux presse préalablement chauffée). Sur 3 éprouvettes testées, d'une épaisseur moyenne 1,35 mm, l' « uniaxial tensile strenght » (UTS) max moyen est de 26,50 MPa.

Le test est réalisé sur un banc de mesure Instron modèle 3342/L2345. Le spécimen est découpé avec un emporte-pièce type bone shape type V cutter tel que décrit dans le standard ASTM D -638-5. La pièce ainsi découpée est introduite par chaque extrémité dans les mords pneumatiques du banc (60psi) en laissant une partie centrale de 2,5 cm. Une vitesse de 25 mm/min est appliquée jusqu'à rupture de la pièce. L' « uniaxial tensile strength » est reporté (max. force / surface sectionnelle). Un « T peel » test est réalisé. Des spécimens de 2 cm x 6 cm sont découpés. L'enduction de P4HB est séparée de l'implant biologique sur une section de 1,5 cm x 2 cm. Les 2 morceaux ainsi séparés sont placés dans les mords pneumatiques (45 psi). La section de l'implant biologique enduite est séparée à une vitesse de 25 mm/min. La « T-peel » force est mesurée sur une largeur normalisée de 20 mm et sur une moyenne de 5 pics (charges). Sur les 3 spécimens testés, l'adhésion était plus forte de sorte que le test n'a pas permis de peler la couche enduite.

### Exemple 9 : exemple de séchage et pressage et caractéristiques des dispositifs médicaux obtenus

Selon les mêmes conditions de tests précédentes, une matrice dermique traitée selon l'exemple 2 est enduite à approximativement 0,03 g/cm² de P4HB puis pressée pendant 60 s à 200 bars et 100°C aboutit à une valeur UTS max moyenne sur 3 éprouvettes de 27,65 MPa. Le « T-peel » test moyen ne peut être calculé car le pelage est incomplet.

### Exemple 10 :

Selon les mêmes conditions de tests précédentes, une matrice dermique traitée selon l'exemple 3 et enduite à approximativement 0,03 g/cm² de P4HB puis pressée pendant 30 s à 100 bars et 100°C, aboutit à une valeur UTS max moyenne sur 3 éprouvettes de 33,07 MPa.

## Revendications

1. Dispositif médical comprenant au moins une matrice biologique acellulaire recouverte partiellement ou totalement par au moins une couche comprenant au moins un polymère, **caractérisé en ce que** la matrice biologique acellulaire a été préparée avant enduction du polymère, de façon à permettre l'adhérence d'une solution de polymère, par un traitement de surface chimique et/ou mécanique et/ou électrochimique et/ou physique.

2. Dispositif médical selon la précédente revendication, **caractérisé en ce qu'**il est constitué par une matrice biologique acellulaire recouverte partiellement ou totalement par une couche comprenant au moins un polymère.

3. Dispositif médical selon la revendication 1, **caractérisé en ce que** la matrice biologique acellulaire est recouverte partiellement ou totalement par au moins deux couches comprenant au moins un polymère.

4. Dispositif médical selon la revendication 1, **caractérisé en ce qu'**il est constitué par une matrice biologique acellulaire recouverte partiellement ou totalement par deux couches comprenant au moins un polymère.

5. Dispositif médical selon l'une des précédentes revendications, **caractérisé en ce que** la matrice biologique acellulaire est d'origine humaine et/ou animale.

6. Dispositif médical selon la précédente revendication, **caractérisé en ce que** la matrice biologique acellulaire d'origine animale est choisie parmi les matrices biologiques acellulaires d'origine porcine, bovine, équine, caprine, de poissons et leurs mélanges.

7. Dispositif médical selon l'une des précédentes revendications, **caractérisé en ce que** la matrice biologique acellulaire est choisie parmi l'une des matrices biologiques suivantes : derme, sous-muqueuse intestinale, aorte, vessie, membrane amniotique, péritoine, péricarde, dure-mère, tendons, os, cartilage et leurs mélanges.

8. Dispositif médical selon l'une quelconque des revendications précédentes, **caractérisé en ce que** le ou les polymère(s) sont choisis parmi les polymères suivants : polyhydroxyalcanoates, poly(glycolides), poly(lactide-co-glycolides), acide polylactique, acide polyglycolique, poly(acide lactique co acides glycolique), polycaprolactones, poly(orthoesters), polyanhydrides, poly(phosphazenes), polyesters, poly(lactide-co-caprolactones), polycarbonates; tyrosine polycarbonates, polyamides, polyesteramides, poly(dioxanones), poly(alkylene alkylates), polyéthers, polyvinyl pyrrolidones, polyuréthanes, polyétheresters, polyacetals, polycyanoacrylates, poly(oxyéthylene), copolymères de poly(oxypropylène), polyacetals, polyketals, polyphosphates, polyphosphoesters, polyalkylène oxalates, polyalkylène succinates, acides polymaléique, chitine, chitosan, et leurs mélanges.

9. Dispositif médical selon l'une quelconque des revendications précédentes, **caractérisé en ce que** le ou les polymère(s) sont choisis parmi les polyhydroxyalcanoates suivants : P4HB, co-polymères, et leurs mélanges.

10. Dispositif médical selon l'une des précédentes revendications, **caractérisé en ce que** la matrice biologique acellulaire :
- présente un taux d'humidité résiduel compris entre 10% et 18%..

11. Procédé de fabrication d'un dispositif médical selon l'une des précédentes revendications, **caractérisé en ce qu'**il comprend la mise en œuvre des étapes suivantes :
- préparation d'une matrice biologique acellulaire de façon à permettre l'adhérence d'une solution comprenant au moins un polymère, par un traitement de surface chimique et/ou mécanique et/ou électrochimique et/ou physique.
- enduction partielle ou totale de la matrice biologique acellulaire avec une solution comprenant au moins un polymère.

12. Procédé de fabrication d'un dispositif médical selon la précédente revendication, **caractérisé en ce que** l'enduction est réalisée à une température inférieure ou égale à la température de dénaturation du collagène.

13. Procédé de fabrication d'un dispositif médical selon la précédente revendication, **caractérisé en ce que** l'enduction est réalisée à une température comprise entre 10 et 60°C.

14. Procédé de fabrication d'un dispositif médical selon l'une des revendications 11 à 13 **caractérisé en ce que** la matrice biologique est séchée de façon à présenter un taux d'humidité résiduel compris entre 10% et 18%.

15. Procédé de fabrication d'un dispositif médical selon l'une des revendications 11 à 14, **caractérisé en ce que** l'enduction est réalisée par enduction par coulage, enduction par vaporisation et enduction par immersion.

16. Procédé de fabrication d'un dispositif médical selon l'une des revendications 11 à 15, **caractérisé en ce que** la solution comprenant au moins un polymère a été préalablement obtenue par solubilisation du ou des polymère(s) dans au moins un solvant polaire.

17. Procédé de fabrication d'un dispositif médical selon la précédente revendication, **caractérisé en ce qu'**après solubilisation du ou des polymère(s), la solution est dégazée et/ou débullée sous vide pour purger le mélange de bulles d'air.

18. Procédé de fabrication d'un dispositif médical selon l'une des revendications 11 à 17, caractérisé en ce la solution de polymère(s) comprend au moins du P4HB solubilisé dans une solution d'acétone.

## Patentansprüche

1. Medizinische Einrichtung, umfassend mindestens eine azelluläre biologische Matrix, die teilweise oder vollständig mit mindestens einer Schicht bedeckt ist, umfassend mindestens ein Polymer, **dadurch gekennzeichnet, dass** die azelluläre biologische Matrix vor einer Beschichtung mit dem Polymer, um die Haftung einer Polymerlösung zu ermöglichen, durch eine chemische und/oder mechanische und/oder elektrochemische und/oder physikalische Oberflächenbehandlung vorbereitet wurde.

2. Medizinische Einrichtung nach dem vorstehenden Anspruch,
**dadurch gekennzeichnet, dass** sie aus einer azellulären biologischen Matrix besteht, die teilweise oder vollständig mit einer Schicht bedeckt ist, umfassend mindestens ein Polymer.

3. Medizinische Einrichtung nach Anspruch 1,
**dadurch gekennzeichnet, dass** die azelluläre biologische Matrix teilweise oder vollständig mit mindestens zwei Schichten bedeckt ist, umfassend mindestens ein Polymer.

4. Medizinische Einrichtung nach Anspruch 1,
**dadurch gekennzeichnet, dass** sie aus einer azellulären biologischen Matrix besteht, die teilweise oder vollständig mit zwei Schichten bedeckt ist, umfassend mindestens ein Polymer.

5. Medizinische Einrichtung nach einem der vorstehenden Ansprüche, **dadurch gekennzeichnet, dass** die azelluläre biologische Matrix von einem Menschen und/oder einem Tier stammt.

6. Medizinische Einrichtung nach dem vorstehenden Anspruch,
**dadurch gekennzeichnet, dass** die azelluläre biologische Matrix, die von einem Tier stammt, aus azellulären biologischen Matrizen ausgewählt ist, die von einem Schwein, einem Rind, einem Pferd, einer Ziege, Fischen und deren Mischungen stammen.

7. Medizinische Einrichtung nach einem der vorstehenden Ansprüche, **dadurch gekennzeichnet, dass** die azelluläre biologische Matrix aus einer der folgenden biologischen Matrizen ausgewählt ist: Lederhaut, intestinaler Submukosa, Aorta, Blase, Amnionmembran, Bauchfell, Herzbeutel, Dura mater, Sehnen, Knochen, Knorpel und deren Mischungen.

8. Medizinische Einrichtung nach einem der vorstehenden Ansprüche, **dadurch gekennzeichnet, dass** das oder die Polymer(e) aus den folgenden Polymeren ausgewählt sind: Polyhydroxyalkanoaten, Poly(glycoliden), Poly(lactid-co-glycoliden), Polymilchsäure, Poly(glycolsäure), Poly(lactid-co-glycolid), Polycaprolactonen, Poly(orthoestern), Polyanhydriden, Poly(phosphazenen), Polyestern, Poly(lactid-co-caprolactonen), Polycarbonaten; Tyrosinpolycarbonaten, Polyamiden, Polyesteramiden, Poly(dioxanonen), Poly(alkylenalkylaten), Polyethern, Polyvinylpyrrolidonen, Polyurethanen, Polyetherestern, Polyacetalen, Polycyanoacrylaten, Poly(oxyethylen), Poly(oxypropylen)-Copolymeren, Polyacetalen, Polyketalen, Polyphosphaten, Polyphosphoestern, Polyalkylenoxalaten, Polyalkylensuccinaten, Poly(maleinsäuren), Chitin, Chitosan und deren Mischungen.

9. Medizinische Einrichtung nach einem der vorstehenden Ansprüche, **dadurch gekennzeichnet, dass** das oder die Polymer(e) aus den folgenden Polyhydroxyalkanoaten ausgewählt ist/sind: P4HB, Copolymeren und deren Mischungen.

10. Medizinische Einrichtung nach einem der vorstehenden Ansprüche, **dadurch gekennzeichnet, dass** die azelluläre biologische Matrix:
- eine Restfeuchtigkeit zwischen 10 % und 18 % aufweist.

11. Verfahren zum Herstellen einer medizinischen Einrichtung nach einem der vorstehenden Ansprüche, **dadurch gekennzeichnet, dass** es die Implementierung der folgenden Schritte umfasst:
- Herstellen einer azellulären biologischen Matrix, um die Haftung einer Lösung, umfassend mindestens ein Polymer, durch eine chemische und/oder mechanische und/oder elektrochemische und/oder physikalische Oberflächenbehandlung zu ermöglichen.
- teilweises oder vollständiges Beschichten der azellulären biologischen Matrix mit einer Lösung, umfassend mindestens ein Polymer.

12. Verfahren zum Herstellen einer medizinischen Einrichtung nach dem vorstehenden Anspruch, **dadurch gekennzeichnet, dass** das Beschichten bei einer Temperatur unter oder gleich der Denaturierungstemperatur des Kollagens erfolgt.

13. Verfahren zum Herstellen einer medizinischen Einrichtung nach dem vorstehenden Anspruch, **dadurch gekennzeichnet, dass** das Beschichten bei einer Temperatur zwischen 10 und 60 °C erfolgt.

14. Verfahren zum Herstellen einer medizinischen Einrichtung nach einem der Ansprüche 11 bis 13, **dadurch gekennzeichnet, dass** die azelluläre biologische Matrix so getrocknet wird, dass sie eine Restfeuchtigkeit zwischen 10 % und 18 % aufweist.

15. Verfahren zum Herstellen einer medizinischen Einrichtung nach einem der Ansprüche 11 bis 14, **dadurch gekennzeichnet, dass** das Beschichten durch Gießbeschichtung, Aufdampfbeschichtung und Tauchbeschichtung erfolgt.

16. Verfahren zum Herstellen einer medizinischen Einrichtung nach einem der Ansprüche 11 bis 15, **dadurch gekennzeichnet, dass** die Lösung, umfassend mindestens ein Polymer, zuvor durch Löslichmachen des Polymers oder der Polymere in mindestens einem polaren Lösungsmittel erhalten wurde.

17. Verfahren zum Herstellen einer medizinischen Einrichtung nach dem vorstehenden Anspruch, **dadurch gekennzeichnet, dass** nach der Löslichmachung des Polymers oder der Polymere die Lösung zum Entfernen von Luftblasen aus der Mischung unter Vakuum entgast und/oder von Blasen befreit wird.

18. Verfahren zum Herstellen einer medizinischen Einrichtung nach einem der Ansprüche 11 bis 17, **dadurch gekennzeichnet, dass** die Polymerlösung mindestens P4HB umfasst, das in einer Acetonlösung gelöst ist.

## Claims

1. A medical device comprising at least one acellular biological matrix covered in whole or in part by at least one layer comprising at least one polymer, **characterized in that** the acellular biological matrix has been prepared prior to polymer coating, so as to allow the adhesion of a polymer solution, by chemical and/or mechanical and/or electrochemical and/or physical surface treatment.

2. The medical device according to the preceding claim, **characterized in that** it consists of an acellular biological matrix covered in whole or in part by a layer comprising at least one polymer.

3. The medical device according to in claim 1, **characterized in that** the acellular biological matrix is covered in part or in whole by at least two layers comprising at least one polymer.

4. The medical device according to in claim 1, **characterized in that** it consists of an acellular biological matrix covered in part or in whole by two layers comprising at least one polymer.

5. The medical device according to one of the preceding claims, **characterized in that** the acellular biological matrix is of human and/or animal origin.

6. The medical device according to the preceding claim, **characterized in that** the acellular biological matrix of animal origin is selected from among the acellular biological matrices of porcine, bovine, equine, caprine, or fish origin, and mixtures thereof.

7. The medical device according to one of the preceding claims, **characterized in that** the acellular biological matrix is selected from one of the following biological matrices: dermis, intestinal submucosa, aorta, bladder, amniotic membrane, peritoneum, pericardium, dura mater, tendons, bones, cartilage, and mixtures thereof.

8. The medical device according to any one of the preceding claims, **characterized in that** the polymer(s) are selected from among the following polymers: polyhydroxyalkanoates, poly(glycolides), poly(lactide-co-glycolides), polylactic acid, polyglycolic acid, poly(lactic acid-co-glycolic acids), polycaprolactones, poly(orthoesters), polyanhydrides, poly(phosphazenes), polyesters, poly(lactide-co-caprolactones), polycarbonates; tyrosine polycarbonates, polyamides, polyesteramides, poly(dioxanones), poly(alkylene alkylates), polyethers, polyvinyl pyrrolidones, polyurethanes, polyether esters, polyacetals, polycyanoacrylates, poly(oxyethylene), copolymers of poly(oxypropylene), polyacetals, polyketals, polyphosphates, polyphosphoesters, polyalkylene oxalates, polyalkylene succinates, polymaleic acids, chitin, chitosan, and mixtures thereof.

9. The medical device according to one of the preceding claims, **characterized in that** the polymer(s) are selected from among the following polyhydroxyalkanoates: P4HB, co-polymers, and mixtures thereof.

10. The medical device according to one of the preceding claims, **characterized in that** the acellular biological matrix:
- has a residual moisture content of between 10% and 18%..

11. A method for manufacturing a medical device according to one of the preceding claims, **characterized in that** it comprises carrying out the following steps:
- preparation of an acellular biological matrix so as to allow the adhesion of a solution comprising at least one polymer, by chemical and/or mechanical and/or electrochemical and/or physical surface treatment.
- partial or total coating of the acellular biological matrix with a solution comprising at least one polymer.

12. The method for manufacturing a medical device according to the preceding claim, **characterized in that** the coating is performed at a temperature lower than or equal to the denaturation temperature of the collagen.

13. The method for manufacturing a medical device according to the preceding claim, **characterized in that** the coating is performed at a temperature between 10 and 60°C.

14. The method for manufacturing a medical device according to one of claims 11 to 13, **characterized in that** the biological matrix is dried so as to have a residual moisture content of between 10% and 18%.

15. The method for manufacturing a medical device according to one of claims 11 to 14, **characterized in that** the coating is carried out by solvent casting, spray coating, or dip coating.

16. The method for manufacturing a medical device according to one of claims 11 to 15, **characterized in that** the solution comprising at least one polymer was obtained beforehand through solubilization of the polymer(s) in at least one polar solvent.

17. The method for manufacturing a medical device according to the preceding claim, **characterized in that,** after solubilization of the polymer(s), the solution is degassed and/or debubbled under vacuum in order to purge the mixture of air bubbles.

18. The method for manufacturing a medical device according to one of claims 11 to 17, **characterized in that** the polymer solution comprises at least P4HB dissolved in an acetone solution.
